# EUROPEAN PATENT APPLICATION

(11) **EP 1 719 521 A1**
(43) Date of publication of application: **08.11.2006**
(21) Application number: 05709431.0
(22) Date of filing: 28.01.2005
(51) Int. Cl.: A61K 38/18, A61P 27/02

(54) **PREVENTIVE AND/OR REMEDY FOR RETINOPATHY**

(30) Priority: 30.01.2004 JP 2004023201
(71) Applicant: Mitsubishi Pharma Corporation, Osaka-shi, Osaka 541-0046 (JP)
(72) Inventor: MACHIDA, Shigeki, Morioka-shi, Iwate 020-0015 (JP); MANO, Tomiya, Ibaraki-shi, Osaka 567-0048 (JP); ISHII, Takehisa, MITSUBISHI PHARMA CORPORATION, Tokyo 103-8405 (JP)
(74) Representative: ter Meer, Nicolaus
(86) International application number: PCT/JP2005/001204
(87) International publication number: WO 2005/072768

(57) **Abstract**

An agent for prophylactic and/or therapeutic treatment of retinopathy, which comprises a hepatocyte growth factor (HGF) as an active ingredient.

## Description

### Technical Field

The present invention relates to a novel medicament useful as an agent for prophylactic and/or therapeutic treatment of retinopathy.

### Background Art

Retina is a layered tissue having a thickness of 0.1 to 0.3 mm that surrounds most of the circumference of an eyeball and has the most important function among eyeball forming units, and a lesion thereof may often result in visual disturbances. Damage or degeneration of retina may be generated by variety of causes, which is generically referred to as retinopathy. Retina has a layered structure consisting of 10 layers. Inner retinal layers (on the vitreous body side) include inner limiting membrane, nerve fiber layer, ganglion cell layer, inner plexiform layer, and inner nuclear layer from the vitreous body side, and intraretinal blood vessels pass through these layers. Outer retinal layers (on the choroid side) include Bruch's membrane, retinal pigment epithelial cell layer, photoreceptor layer, outer limiting membrane, outer nuclear layer, and outer plexiform layer from the choroid side, and no blood vessel passes through this region.

Many diseases are known as retinopathy, and from a viewpoint of types of the onset, the diseases are mainly classified into those of a type resulting from damage or degeneration of the inner retinal layers and those of a type resulting from damage or degeneration of the outer retinal layers. The former class of diseases include hypertensive retinopathy and diabetic retinopathy in which retinopathy is resulted from angiopathy or neovascularization in the inner retinal layer. The latter class of diseases include macular degeneration, retinitis pigmentosa and the like in which retinopathy is resulted from pathological changes of the retinal pigment epithelial layer or photoreceptor layer. As described above, the diseases resulting from damage or degeneration of the inner retinal layers and those resulting from damage or degeneration of the outer retinal layers are clearly distinguished.

Incidence of retinopathy has been drastically increasing due to progressive increase of elderly population and change of dietary life (increase of diabetes patients), and poor prognosis or unsatisfactory QOL (quality of life) of retinopathy causes problems. Various therapies have been developed for the treatment of retinopathy. For example, surgical operations applying laser photocoagulation have been effectively performed in some class of patients. Basically, however, the treatment depends on symptomatic therapies involving medical control of primary diseases (hypertension, diabetes and the like), and no effective therapeutic agent has been available to date. Further, for age-related macular degeneration, of which patients have been sharply increasing, no effective surgical therapy or therapeutic agent has been available so far, and therefore, an excellent therapeutic agent is strongly desired.

Under the circumstances, studies have been made on chemotherapies by using interferons, anti-vascular endothelial cell growth factor (VEGF) monoclonal antibodies, steroids and the like for a purpose of inhibition of neovascularization that accompanies retinopathy. For purposes of promotion of the growth and protection of optic nerve, studies have been also made on basic-fibroblast growth factor (bFGF), ciliary neurotrophic factor (CNTF), lens epithelium-derived growth factor (LEDGF) and hepatocyte growth factor (hereinafter also referred to as "HGF") in animal models. However, none of these therapies has been established as a clinical therapy.

HGF, having a superior neovascularization action, has been developed as a therapeutic agent for obstructive arteriosclerosis. Treatment of obstructive arteriosclerosis by using an HGF gene has also been developed clinically. As for studies of effectiveness of HGF on retinopathy, a patent application was filed relating to a use of a partial fragment of HGF for retinopathy having an antagonistic activity (Patent document 1). According to the invention, however, HGF is considered to act as a retinopathy aggravating factor, and the invention does not provides an agent for prophylactic and/or therapeutic treatment of retinopathy by using HGF, per se.

An application of HGF, per se, to a retinal ischemia-reperfusion model has also been reported, which model is one of the retinopathy models (Non-patent document 1). In this model, physiological saline is injected into the rat eyeball to increase ocular tension and thereby cause retinal ischemia. On the basis of the type of onset of diseases, the model can be regarded as that of a retinal disease resulting from damage to blood vessels or disturbance of blood flow in the inner retinal layers, such as hypertensive retinopathy, retinopathy of prematurity, glaucoma, and retinal vascular occlusion. In this model, HGF exhibited an effect of protecting nerve cells in the inner retinal layers such as the ganglion cell layer and inner nuclear layer. However, no effect of HGF has been shown on damage or degeneration of retina pigment epithelial cells and photoreceptors in the outer retinal layers.

Further, a report of gene therapies by using neovascularization factors (Patent document 2) includes a description of HGF as an example of the neovascularization factors, and effectiveness of HGF for therapeutic treatment of age-related macular degeneration is described. However, what is described as examples is an effect of VEGF on damage to the inner retinal layers, and no effect on damage to the outer retinal layers is shown.

As mentioned above, it has not been known that HGF is effective for prophylactic and/or therapeutic treatment of retinopathy resulting from degeneration of the outer retinal layers such as macular degeneration or retinitis pigmentosa. As for retinitis pigmentosa, in particular, the Royal College of Surgeon (RCS) rat is known as a model of retinitis pigmentosa which has the same gene mutations as those in human diseases. It is important to conduct evaluation by using this model animal to examine applicability of a drug to treatment of retinitis pigmentosa.

Calcium antagonists, which are known to improve retinal circulation and show efficacy in inner retinal layer models, are not necessarily effective in outer retinal layer models (Non-patent document 2). Accordingly, effect on retinopathy such as macular degeneration and retinitis pigmentosa resulting from degeneration of the outer retinal layers cannot be predicted only by evaluation in an inner retinal layer model such as the ischemia-reperfusion model.

HGF was discovered as a potent growth promoting factor acting on mature hepatocytes, and the gene thereof was cloned (Non-patent document 3). Subsequent studies have revealed that HGF is associated in vivo with wound healing in the kidney, lung, stomach, duodenum and skin, and that the c-Met proto-oncogene codes for an HGF receptor (Non-patent documents 4 and 5).

At present, HGF is considered to be a factor acting on many kinds of tissue repairs and organ regenerations via this receptor (Non-patent documents 6 and 7).
Patent document 1: International Patent Publication WO01/44294
Patent document 2: U.S. Patent Application No. 2003/0053989
Non-patent document 1: Shibuki, H et al., Invest. Ophthalmol. Vis. Sci., Vol. 43, 528-536 (2002)
Non-patent document 2: Bush et al., Invest. Ophthalmol. Vis. Sci., Vol. 41, 2697-2701 (2000)
Non-patent document 3: Biochem. Biophys. Res. Commun., Vol. 163, 967 (1989)
Non-patent document 4: Science, Vol. 251, 802-804 (1991)
Non-patent document 5: Oncogene, Vol. 6, 501-504 (1991)
Non-patent document 6: Jikken Igaku (Experimental Medicine), Vol. 10, 144-153 (1992)
Non-patent document 7: Domyakukoka (Arteriosclerosis), Vol. 23, 683-688 (1996)

### Disclosure of the Invention

### Object to be Achieved by the Invention

An object of the present invention is to provide a novel agent for prophylactic and/or therapeutic treatment of retinopathy.

### Means for Achieving the Object

The inventors of the present invention conducted various researches to achieve the forgoing object. As a result, they found that administration of HGF is effective for retinopathy, in particular, diseases resulting from damage or degeneration of the outer retinal layers such as macular degeneration and retinitis pigmentosa. The present invention was achieved on the basis of these findings.

The gist of the present invention is constituted by:
1. an agent for prophylactic and/or therapeutic treatment of retinopathy resulting from damage and/or degeneration of the outer retinal layers, which comprises a hepatocyte growth factor as an active ingredient;
2. an agent for prophylactic and/or therapeutic treatment of a disease selected from macular degeneration and retinitis pigmentosa, which comprises a hepatocyte growth factor as an active ingredient; and
3. an agent for prophylactic and/or therapeutic treatment of retinopathy, which comprises a hepatocyte growth factor as an active ingredient.

### Brief Description of the Drawings

[Fig. 1] Fig. 1 shows waveforms in electroretinograms of normal SD rats and retinal light-damaged rats (HGF-administered group and solvent-administered group) under scotopic (Fig. 1A) and photopic (Fig. 1B) conditions.
[Fig. 2] Fig. 2 shows light intensity-ERG response curves of normal SD rats and retinal light-damaged rats (HGF-administered group and solvent-administered group) under scotopic (Fig. 2A) and photopic (Fig. 2B) conditions.
[Fig. 3] Fig. 3 shows waveforms in electroretinograms of 24-day-old RCS rats before onset (baseline) and 70-day-old RCS rats (HGF-administered group and solvent-administered group) under scotopic (Fig. 3A) and photopic (Fig. 3B) conditions.
[Fig. 4] Fig. 4 shows light intensity-ERG response curves of 24-day-old RCS rats before onset (baseline) and 70-day-old RCS rats (HGF-administered group and solvent-administered group) under scotopic (Fig. 4A) and photopic (Fig. 4B) conditions.
[Fig. 5] Fig. 5 shows retinal tissue micrographs of non-damaged normal SD rats and light-damaged rats (HGF-administered group and solvent-administered group). The nucleic numbers of rod and cone per unit area in the retinal tissue micrograph of each group, and thicknesses of the outer nuclear layer and the photoreceptor layer (rod and cone layer) were measured.
[Fig. 6] Fig. 6 shows retinal tissue micrographs of 24-day-old RCS rats (baseline) before onset and 70-day-old RCS rats (HGF-administered group and solvent-administered group). Further, the nucleic numbers of rod and cone per unit area in the retinal tissue micrograph of each group and thicknesses of the outer nuclear layer and the photoreceptor layer (rod and cone layer) were measured.
[Fig. 7] Fig. 7 shows effect of HGF on sodium iodate-induced retinal pigment epithelial cells.
[Fig. 8] Fig. 8 shows survival rates of retinal pigment epithelial cells after 1 day of HGF-added culture in Example 3.
[Fig. 9] Fig. 9 shows effect of CNTF on sodium iodate-induced retinal pigment epithelial cells.
[Fig. 10] Fig. 10 shows survival rates of retinal pigment epithelial cells after 1 day of CNTF-added culture in Example 3.

### Best Mode for Carrying out the Invention

HGF contained in the agent for prophylactic and/or therapeutic treatment of the present invention is a known substance, and any HGF prepared by any of various methods can be used so long as the substance is purified to an extent satisfactorily usable as a medicament. For example, an HGF can be obtained by culture of primary cultured cells or cells of an established cell line that produce HGF, isolation of the product from the culture supernatant or the like and successive purification. Alternatively, a gene encoding an HGF can be incorporated into an appropriate vector by means of a genetic engineering technique, and then the vector is introduced into a suitable host for transformation, and an desired recombinant HGF can be isolated from a culture supernatant of the transformant. The aforementioned host cells are not particularly limited, and various host cells conventionally used in genetic engineering techniques can be used such as *Escherichia coli,* yeast, baculovirus (polyhedrosis virus of arthropods)-insect cells and animal cells (see, Biochem. Biophys. Res. Commun. Vol. 175, 660 (1991); Japanese Patent No. 2577091 (corresponding European Patent Application No. 412557) and the like).

Each transformant introduced with the recombinant vector is cultured in a suitable medium. The medium may contain a carbon sources, nitrogen sources, inorganic substances, vitamins, and serum necessary for the growth of the transformant, and an agent used for resistance screening and the like. Specifically, examples include LB medium (Nacalai Tesque Inc.) and the like when *Escherichia coli* is used as a host of the transformant, YPD medium (Genetic Engineering, 1, 117, Plenum Press [1979]) and the like for yeast, Ham-12 medium, MEM medium, DMEM medium, RPMI1640 medium (SIGMA) and the like containing 20% or lower of fetal calf serum for insect cells or animal cells, and the like. Further, culture temperature, CO₂ concentration and culture time can be suitably selected depending on the host, recombinant vector and the like. Further, if necessary, the culture medium is aerated and agitated. Medium compositions and culture conditions other than those mentioned above may also be employed so long as the introduced host can grow, and the protein encoded by the inserted HGF polynucleotide can be produced.

As for methods for collecting the transformed product obtained by the culture as described above, when the host is a cell, for example, a method may be employed in which the cells are isolated from the culture by centrifugation or the like to collect the product as cells or a culture supernatant and the like. Examples of the method for extracting the recombinant protein from the recovered cells include ordinarily used known methods.

HGF can also be prepared by using a known cell-free protein synthesis system or the like. Specifically, as the cell extract used for the cell-free protein synthesis system, known cell extracts, for example, cell extracts of microorganisms such as *Escherichia coli;* plant germs, rabbit reticulocytes and the like are used. Those commercially available can be used, or the extracts can be prepared by methods known per se. Specifically, *Escherichia coli* extracts can be prepared according to the methods described in Pratt, J. M. et al., Transcription and Translation, Hames, 179-209, B. D. and Higgins, S. J., et al., IRL Press, Oxford (1984).

In an HGF obtained as described above, one or more amino acid residues in the amino acid sequence thereof may be substituted, deleted and/or added so long as the HGF has actions substantially identical to those of natural HGF, and similarly, a sugar chain may be substituted, deleted and/or added.

As the agent for prophylactic and/or therapeutic treatment of the present invention, HGF alone may be administered, or a pharmaceutical composition in the form of pharmaceutical preparation can be prepared together with suitable pharmaceutical additives and administered. A dosage form of the pharmaceutical composition is not particularly limited so long as the form is commonly used for parenteral administration. Eye drops and ophthalmic ointments are preferred from a viewpoint of the target of the therapeutic or prophylactic treatment. When administration to the eyeball or systemic administration is required, an ampoule for injection, lyophilized powder for injection or the like can be used. Various dosage forms can be prepared according to conventionally used techniques by using known pharmaceutical additives available to those skilled in the art such as diluents and additives.

For example, an eye drop is prepared by mixing an effective amount of the aforementioned purified HGF with an isotonic agent such as sucrose and a preservative and diluting the mixture with distilled water for injection to an appropriate concentration. A lyophilized powder for injection can be produced by a conventional method by dissolving an effective amount of the aforementioned purified HGF in a diluent and adding an excipient, stabilizer, preservative, soothing agent, pH modifier and the like, if necessary. Further, an ampoule for injection can be prepared by dissolving an effective amount of the aforementioned HGF in a diluent, adding additives such as a dissolving aid, buffer, isotonic agent, stabilizer, preservative, soothing agent and pH modifier, if necessary, and then sterilizing the mixture by ordinary sterilization, aseptic filtration or the like. Since HGF may sometimes be inactivated during the process of thermal sterilization, the sterilization method should be suitably selected.

In addition to the aforementioned parenteral administration, the agent for prophylactic and/or therapeutic treatment of retinopathy of the present invention can also be used as pharmaceutical preparations formulated in dosage forms suitable for oral administration, inhalation, or external use in a solid form such as a tablet, a granule, a capsule and a powder or in a liquid form such as a solution, a suspension, a syrup, an emulsion, and a lemonade by using pharmaceutically acceptable carriers and the like. If necessary, the aforementioned formulations may be mixed with adjuvants, stabilizers, skin wetting agents or other conventional additives.

Doses of the aforementioned various pharmaceutical preparations may vary depending on the route of administration, a type of a disease, symptoms, a body weight or an age of a patient, as well as a type of the medicament to be applied. In general, a dose of about 1 to 200 mg or more per day for a single patient can be administered. HGF contained as an active ingredient is preferably applied to prophylactic and/or therapeutic treatment of retinopathy at an average dose of about 5 to 100 mg for a single administration.

The medicament of the present invention may be mixed with an active ingredient of another medicament having a pharmacological action similar to or other than that of the HGF preparation of the present invention. Further, substances such as sulfated polysaccharides and derivatives thereof, e.g., heparin and dextran sulfate, may be added which are known to enhance the hepatocyte growing action of HGF as the active ingredient of the medicament of the present invention (Japanese Patent Unexamined Publication (Kokai) No. 5-301824 (corresponding to European Patent Application No. 517182)).

Further, according to the present invention, an HGF gene can be used as an agent for gene therapy to achieve prophylactic and/or therapeutic treatment of retinopathy, or appropriate cells are introduced with an HGF gene and then the cells can be used as an agent for cell therapy to transplant the cell into a tissue. For example, an HGF according to the present invention is mixed in a lipofection reagent and the resulting mixture is administered in vivo to maintain a local HGF concentration at a level necessary for prophylactic and/or therapeutic treatment of retinopathy. Although it depends on severity of a disease, responses of a living body and the like, administration of HGF according to the present invention may be conducted with a suitable dose, an administration method, and frequency for a period until effectiveness of the prophylactic and/or therapeutic treatment is recognized or amelioration of the pathological condition is achieved.

### Examples

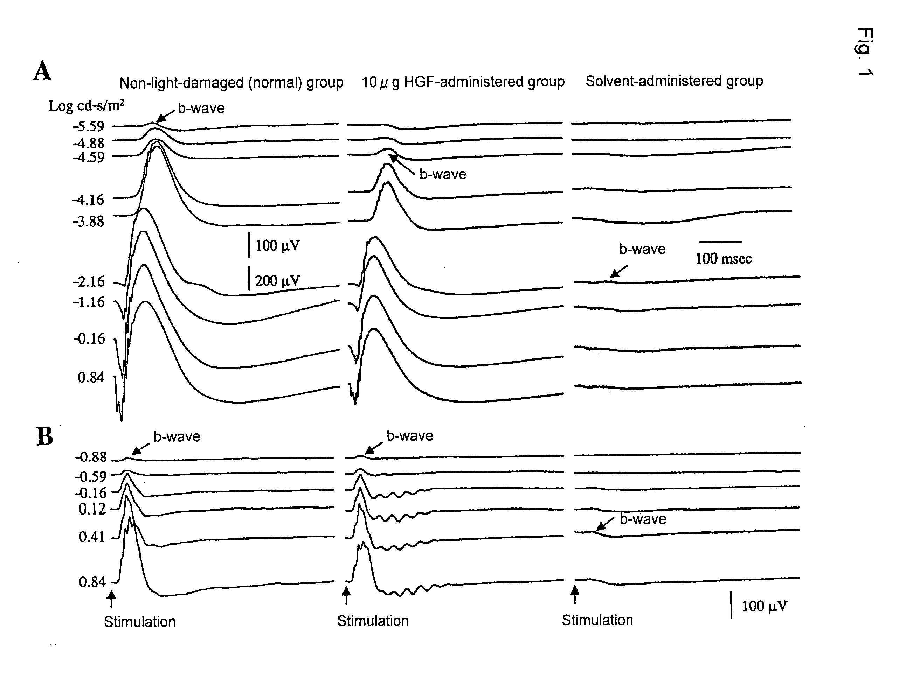

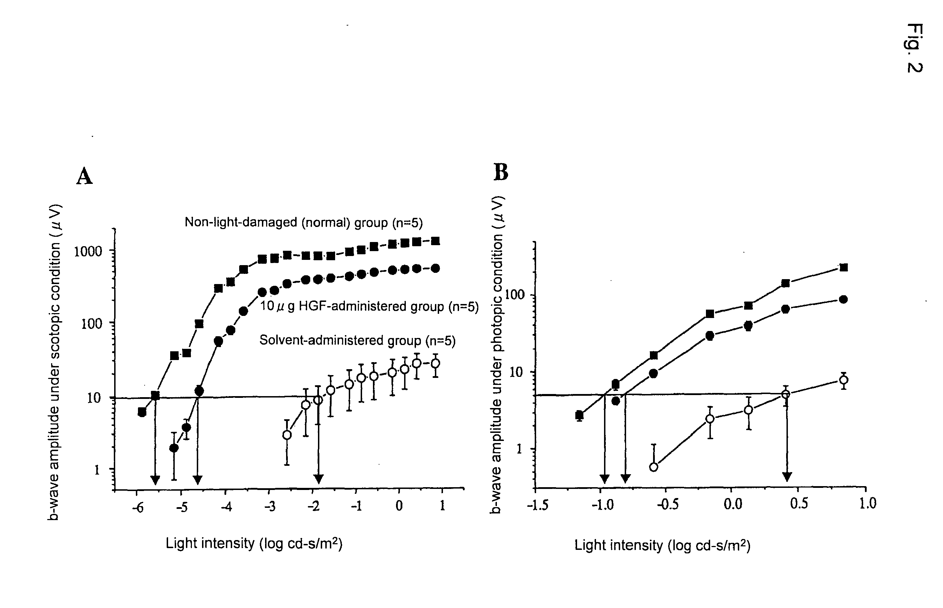

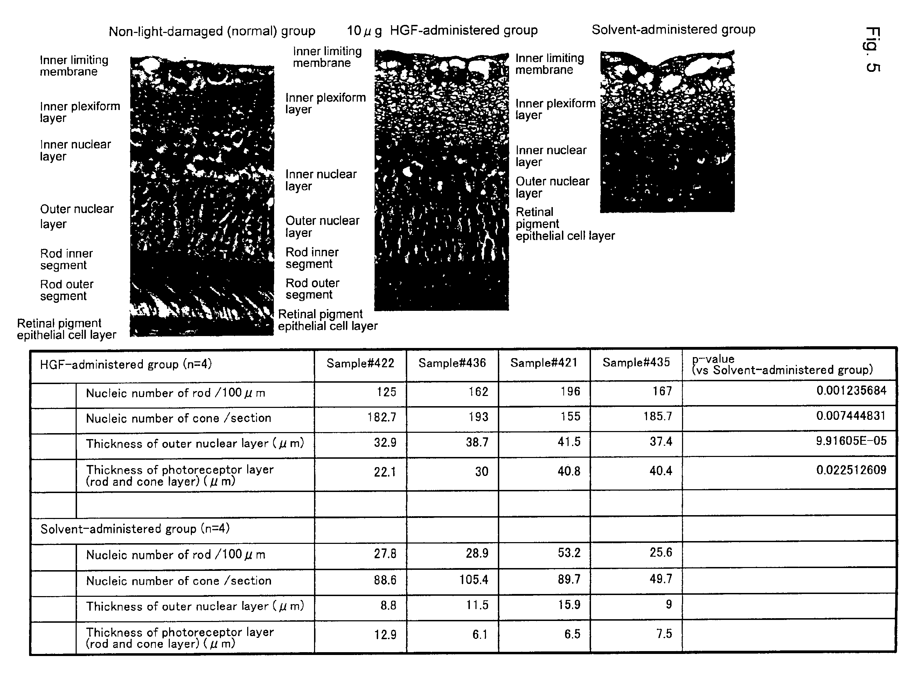

The present invention will be further explained with reference to the following examples. However, the scope of the present invention is not limited to the examples. Fundamental methods applied in the examples were according to the methods described in Machida et al. Invest. Ophthalmol. Vis. Sci. Vol. 42, 1087-1095 (2001).
(Example 1) Effect of administration of HGF in rat light-damaged model
(Preparation of light-damaged model and administration of HGF)
Retinal light-damaged rats were prepared by irradiating 8-week old male Sprague-Dawley rats (n = 19) with 3,000 lux of white light for 72 hours. Two days before the light irradiation, HGF (10 µg/2 µl) dissolved in phosphate-buffered saline (pH 7.4) containing heparin (5 µg/µl, Sigma H5248) was intravitreously injected to the right eyes of the rats, and the same amount of the solvent was given to the left eyes as a control group. Further, rats of the same age in week which were not subjected to the light irradiation were also analyzed for comparison.
(Confirmation of effect)
On the 14th day after the light irradiation, electroretinograms (ERG) were recorded under scotopic and photopic conditions. The light stimulation intensity was increased from the threshold of the scotopic threshold response in 0.27, 0.28 or 0.43-log-unit. The duration of light stimulation was 10 µsec, and the maximum luminance was 0.84 log cd-s/m². The photopic ERG were recorded with a white rod-suppressing background of 34 cd/m², and the rats were light-adapted for at least 10 minutes with the same background light before the photopic recording. Light intensity-ERG response curves were prepared for the non-damaged group (n = 5), light-damaged/HGF-administered group (n = 5), and light-damaged/solvent-administered group (n = 5), and the maximum amplitudes and thresholds of ERG b-waves of HGF-treated eyes under the scotopic and photopic conditions were compared with those of the control group and the non-damaged group. Further, after recording ERG, a retinal tissue sample of each rat was prepared, the nucleic numbers of rod and cone remaining in the outer nuclear layer and thicknesses of the outer nuclear layer as well as the photoreceptor layer (rod and cone layer) were measured. The retinal tissue was fixed with 2.5% glutaraldehyde for 2 hours, then with 5% formalin buffer overnight, and paraffin-embedded to prepare a sectional sample having a thickness of 3 µm, which was further stained with hematoxylin-eosin (HE). The number of rods was obtained by counting the number of nuclei remaining in 100 µm of the retina photoreceptor layer and the outer nuclear layer at 20 sites (400-µm intervals) of the retina and calculating the average value. The number of cones was obtained by counting the number of cone nuclei remaining in one retinal section.
(Results)
In retinal light-damaged rats, the maximum amplitudes and thresholds of the ERG b-wave of the HGF-treated eyes remained significantly more favorable under scotopic and photopic conditions compared with the control group (b-wave maximum amplitude: p < 0.0005, b-wave threshold: p < 0.05, Figs. 1 and 2). Further, the nucleic numbers of rod and cone in the HGF-treated eyes remained in a significantly larger number compared with the control group (p < 0.005, p < 0.05). The thickness of the retinal outer nuclear layer as well as the thicknesses of the photoreceptor layer (rod and cone layer) were also significantly maintained in the HGF-treated eyes (p < 0.0005, p < 0.05, Fig. 5). Tissue micrographs of these samples are also shown in Fig. 5. In the retina tissue samples of the light-damaged model rats, a clear protective effect was recognized, and no adverse reaction such as abnormal neovascularization was recognized (Fig. 5).
(Example 2) Effect of administration of HGF in RCS rat model
(RCS rats and administration of HGF)
HGF was given to each of 24-day-old Royal College of Surgeon (RCS) rats, which is an animal model for hereditary photoreceptor degeneration, in the same manner as in Example 1. After completion of the administration, rats were returned to the original colony and fed under a standard light condition (5 lux in the daytime and dark during the night in the feeding cage).
(Confirmation of effect)
RCS rats were given with HGF or the solvent, and then electroretinograms (ERG) were recorded at the age of 70 days under scotopic and photopic conditions. The measurement conditions and the like were as described in Example 1. As a baseline for administration of HGF, 24-day-old untreated RCS rats were also analyzed for comparison. Almost no retinal degeneration was observed in the RCS rats at the age of 24 days.

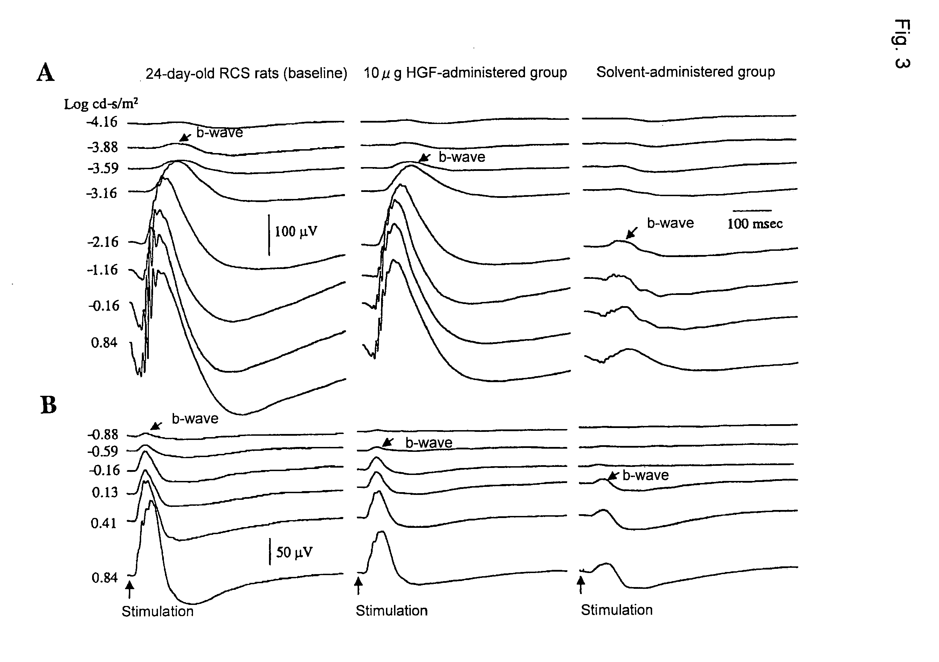

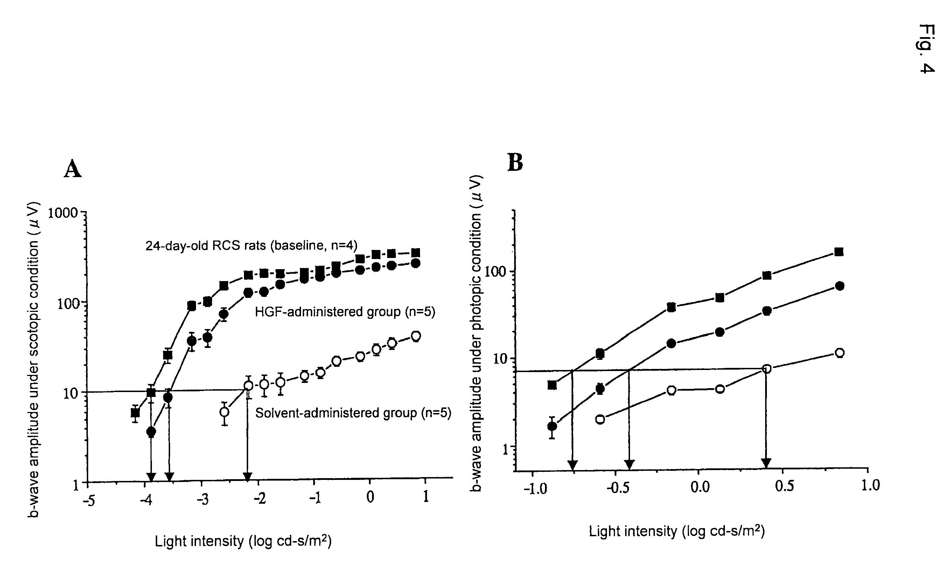

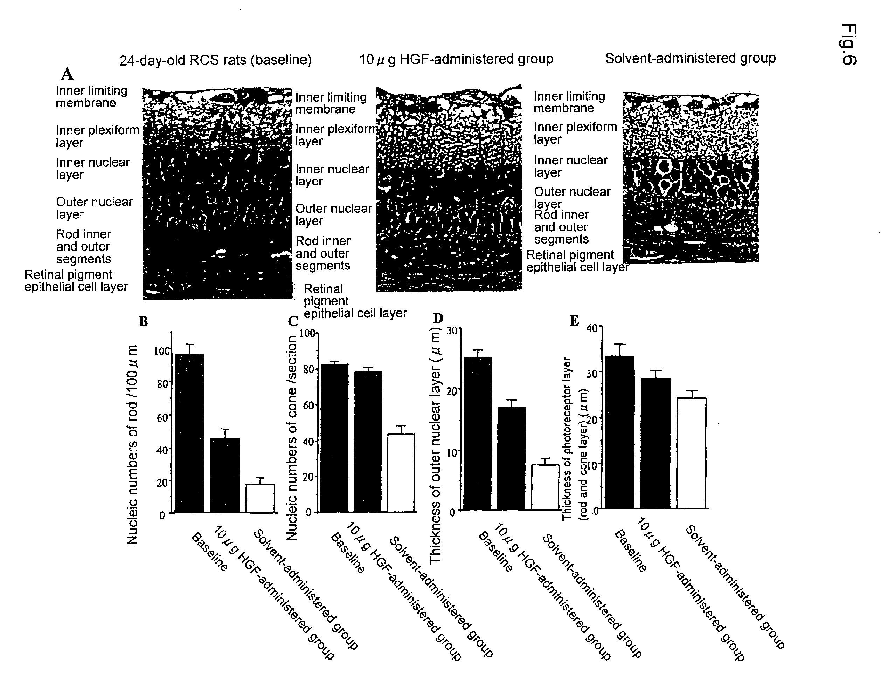

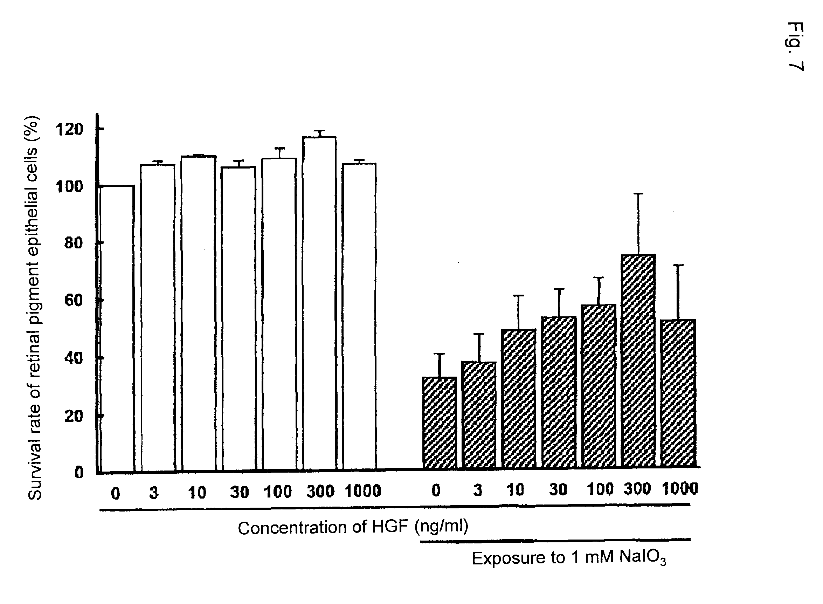

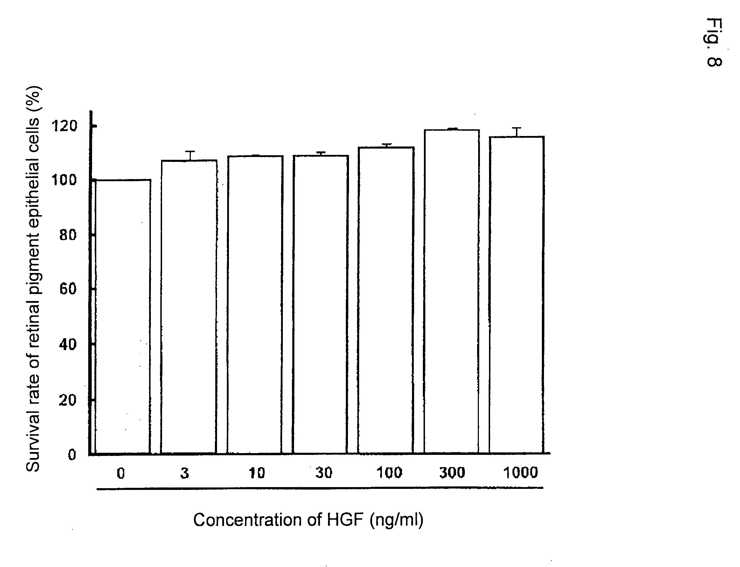

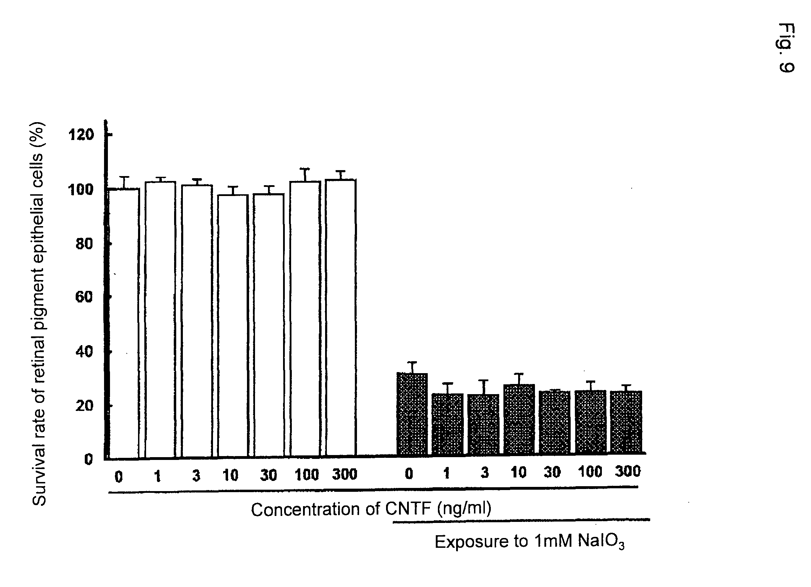

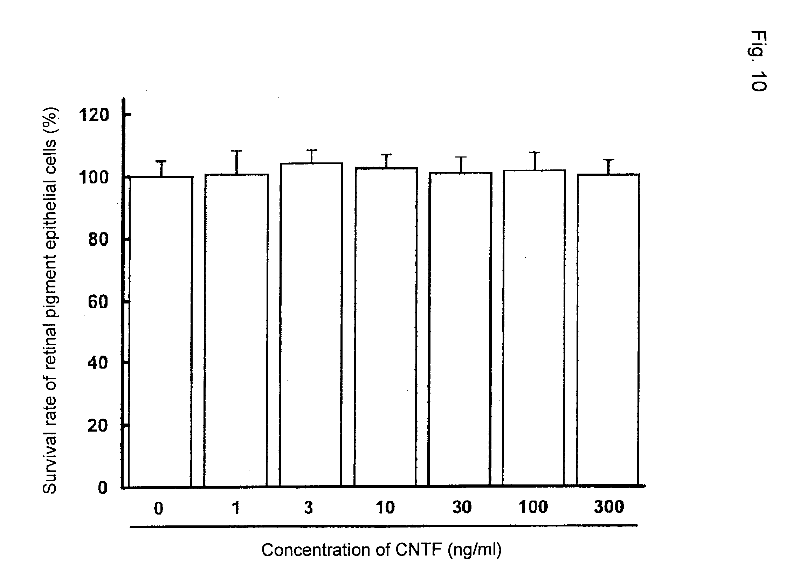

Light intensity-ERG reaction curves were prepared for the 24-day-old RCS rat group (baseline, n = 4), HGF-administered group (n = 5) and solvent-administered group (n = 5), and the maximum amplitudes and thresholds of the ERG b-waves of HGF-treated eyes under scotopic and photopic conditions were compared with those of the control group and the 24-day-old (baseline) group. Further, after recoding ERG, a retina tissue sample of each rat was prepared in the same manner as in Example 1, and the nucleic numbers of rod and cone remaining in the outer nuclear layer and the thicknesses of the retinal outer nuclear layer as well as the photoreceptor (rod and cone layer) were measured.
(Results)
In the RCS rats, the maximum amplitudes and thresholds of the ERG b-wave of the HGF-treated eyes remained significantly more favorable under scotopic and photopic conditions compared with the control group (b-wave maximum amplitude: p < 0.0005, b-wave threshold: p < 0.05, Figs. 3 and 4). Further, the nucleic numbers of rod and cone in the HGF-treated eyes remained significantly larger compared with the control group. The thickness of the retinal outer nuclear layer as well as the thicknesses of the photoreceptor layer (rod and cone layer) were also significantly maintained in the HGF-treated eyes (p < 0.05, Fig. 6). Tissue micrographs of these samples are also shown in Fig. 6. Even at the age of 70 days (46 days after the HGF administration), a clear retina protection effect was recognized in the tissue micrographs, and no adverse reaction such as abnormal neovascularization was recognized.
(Conclusion)
HGF protects photoreceptors (rods and cones) in the retinal outer nuclear layer, which are the most important for visual recognition, from degeneration in the retinal light-damaged model and hereditary retina degeneration model.
(Example 3) Effect on sodium iodate-induced retinal pigment epithelial cells
Retinal pigment epithelial cells were isolated from 7- to 8-day old Long Evans rats and cultured in a 75-cm² culture flask until the cells reached confluent. The retinal pigment epithelial cells digested with trypsin were inoculated on a 96-well culture plate in the presence of HGF or CNTF, cultured overnight, then added with 1 mM sodium iodate and cultured for 2 days. Then, the ratio of survived cells was measured by MTS assay. The results are shown in Figs. 7 to 10.

From these results, it was found that HGF successfully protected retinal pigment epithelial cells, whilst CNTF failed to protect the cells.

### Industrial Applicability

The medicament of the present invention comprising the hepatocyte growth factor as an active ingredient is useful as a superior agent for prophylactic and/or therapeutic treatment of retinopathy.

Although the present invention is explained in detail by referring to specific embodiments thereof, it is apparent to those skilled in the art that various alterations and modifications are achievable without departing from the spirit and scope of the present invention.

This application is based on Japanese Patent Application No. 2004-023201 filed on January 30 2004, which whole disclosure is incorporated herein by reference.

## Claims

1. An agent for prophylactic and/or therapeutic treatment of retinopathy resulting from damage and/or degeneration of the outer retinal layers, which comprises a hepatocyte growth factor as an active ingredient;

2. An agent for prophylactic and/or therapeutic treatment of a disease selected from macular degeneration and retinitis pigmentosa, which comprises a hepatocyte growth factor as an active ingredient.

3. An agent for prophylactic and/or therapeutic treatment of retinopathy, which comprises a hepatocyte growth factor as an active ingredient.
